(19) **Europäisches Patentamt**

**European Patent Office**

**Office européen des brevets**

(11) **EP 1 053 759 B1**

(12) **EUROPÄISCHE PATENTSCHRIFT**

(45) Veröffentlichungstag und Bekanntmachung des Hinweises auf die Patenterteilung:
**16.04.2003 Patentblatt 2003/16**

(51) Int Cl.$^7$: **A61M 1/16**

(21) Anmeldenummer: **00119042.0**

(22) Anmeldetag: **03.11.1995**

(54) **Verfahren zur Steuerung einer Vorrichtung zur partiellen Dialysatsammlung**

Method for controlling a device for partial dialysate collection

Méthode de réglage d'un appareil pour la collection partielle de dialysat

(84) Benannte Vertragsstaaten:
**DE ES FR GB IT SE**

(30) Priorität: **12.11.1994 DE 4440556**

(43) Veröffentlichungstag der Anmeldung:
**22.11.2000 Patentblatt 2000/47**

(62) Dokumentnummer(n) der früheren Anmeldung(en) nach Art. 76 EPÜ:
**95117313.7 / 0 711 569**

(73) Patentinhaber: **Fresenius Medical Care Deutschland GmbH**
**61350 Bad Homburg v.d.H. (DE)**

(72) Erfinder: **Polaschegg, Hans-Dietrich, Dr.**
**9231 Köstenberg (AT)**

(74) Vertreter: **Luderschmidt, Schüler & Partner GbR Patentanwälte,**
**John-F.-Kennedy-Strasse 4**
**65189 Wiesbaden (DE)**

(56) Entgegenhaltungen:
**EP-A- 0 621 046         WO-A-94/08641**
**DE-A- 3 436 748         FR-A- 2 692 983**

**Beschreibung**

Die Erfindung betrifft ein Verfahren zur Ermittlung der Effektivität einer Hämodialysebehandlung bzw. der bei einer Hämodialysebehandlung entfernten Urämietoxine.

Die chronische Hämodialysebehandlung ist die am meisten verbreitete Nierenersatztherapie. Ungefähr 500000 Patienten leben mit dieser Therapie, da bessere Alternativen wie z.B. die Transplantation von Kadavernieren für sie nicht zur Verfügung stehen.

Die Behandlungskosten, die insgesamt etwa 50000 US$ pro Jahr und Patient ausmachen haben zu einer enormen Belastung der Krankenversicherungen und des Gesundheitssystems geführt. Nur ein Teil der Kosten ist durch die Hämodialysebehandlung direkt hervorgerufen, der größere Teil fällt auf sonstige Behandlungen, Krankenhausaufenthalte, Blutanschlußprobleme und Transport zur Dialysestation. Der Druck zur Kosteneinsparung trifft jedoch die eigentliche Dialysebehandlung, da diese schematisiert ist und von großen Organisation durchgeführt wird.

Der Druck auf die Behandlungskosten hat zu einer Verringerrung der Hämodialysezeit und häufig auch der Hämodialysequantität geführt. In den USA, wo etwa 92 % der Behandlungen über eine staatliche Organisation (Medicare) finanziert werden und eine umfangreiche statistische medizinische Datenbasis besteht, beobachtete man in den 80er Jahren einen Rückgang der Überlebenswahrscheinlichkeit von Patienten, der auf eine Verringerrung der Dialysedosis pro Behandlung zurückgeführt wurde. Auf Grund dieser Erkenntnis wurden Forschungsarbeiten intensiviert, mit dem Ziel, die minimal nötige und die optimale Dialysedosis zu ermitteln. Dadurch ausgelöst begann auch eine intensivere Forschung nach Methoden, die verabreichte Dialysedosis zuverlässig und kostengünstig zu ermitteln. Die Betonung "kostengünstig" ist deshalb wichtig, da der auslösende Grund ja gerade die Kosteneinsparung war. Eine Meßmethode, welche die Kosten wesentlich erhöht, kann den Zweck, eine ausreichende Dialysebehandlung bei gleichem Kostenaufwand zu garantieren, nicht erfüllen, da dann der Aufwand für die eigentliche Behandlung weiter verringert werden muß.

Aufgabe dieser Erfindung ist es, ein Verfahren anzugeben, das mit minimalem Hardwareaufwand im Dialysegerät die Ermittlung der Menge an entfernten Urämietoxinen ermöglicht, ohne Blutproben zu erfordern.

Ein anerkanntes Verfahren zur Beurteilung der Dialyseeffektivität ist die Harnstoffkinetik. Die Reduktion der Plasmakonzentration von Harnstoff von Beginn zum Ende der Hämodialyse wird dabei als Maß für die Effektivität herangezogen. Es ist allgemein bekannt, daß Harnstoff nicht akut toxisch ist und in diesem Fall lediglich als Referenzsubstanz zur Messung herangezogen wird.

Die Harnstoffkinetikmethode ist z.B. in der Arbeit von: "Sargent JA, Gotch FA. Principles and Biophysics of Dialysis. Mäher JF, editors. Replacement of Renal Function by Dialysis, 3rd ed.. Kluwer Academic Publishers, 1989:87-143" ausführlich beschrieben. Die Standardmethode geht von einer gleichmäßigen Verteilung von Harnstoff im Ganzkörperwasser aus und wird deshalb 1-pool Modell genannt. Eine vollständige Messung umfaßt mindestens drei Blutproben. Je eine zu Beginn und Ende einer Behandlung und eine weitere vor Beginn der nächsten. Die Harnstoffkonzentration fällt während der Dialyse exponentiell ab. Aus den genannten beiden ersten Harnstoffmessungen zu Beginn und Ende der Dialyse kann nun der Exponentialkoeffizient diese Abfalls errechnet werden, der durch K*T/V charakterisiert ist. K ist dabei die effektive Harnstoffclearance, T die Dialysezeit und V das Harnstoffverteilungsvolumen, gewöhnlich mit dem Ganzkörperwasser gleichgesetzt. Durch klinische Beobachtung wurde gefunden, daß KT/V mindestens 1 sein muß.

Aus der 2. und 3. Messung der beschriebenen Meßserie läßt sich die Menge des durch den Metabolismus erzeugten Harnstoffs bestimmen, aus dem wiederum, wie in "Replacement of Renal Function by Dialysis" beschrieben, die Menge an umgesetzten Protein (Protein Catabolic Rate) berechnet werden kann. Diese ist von Bedeutung, da man festgestellt hat, daß die Überlebenswahrscheinlichkeit sehr stark vom Plasmaalbumingehalt und dieser wiederum von der Proteinaufnahme abhängt.

Die Verkürzung der Dialysezeit bei gleichzeitiger Erhöhung der Clearance hat dazu geführt, daß das oben beschriebene 1-pool Modell den tatsächlichen Vorgang nur mehr ungenügend beschreibt. Der Fehler kann dabei bis zu 40% betragen. Man ist daher zu 2-pool Modellen übergegangen, zu deren Charakterisierung wenigstens 3 intradialytische Blutproben erforderlich sind, wobei die letzte etwa 30 bis 60 Minuten nach Beendigung der Hämodialyse genommen wird Dies hat die Charakterisierung der Behandlungsquantität weiter erschwert.

Seit Beginn der Hämodialysebehandlungstechnik hat man versucht, die entfernten Urämietoxine in der Austauschflüssigkeit zu messen. Dazu wurde die gesamte Dialysierflüssigkeit aufgefangen und die Menge bestimmt (siehe z.B. Lankhorst BJ, Ekllis P, Nosse C, Malchesky P, Magnusson MO. A Practical Guide to Kinetic Modeling Using the Technique of Direct Dialysis Quantification. Dialysis & Transplantation 1983;12: 694-706). Die Konzentration von Harnstoff und anderen Urämietoxinen in einer Probe dieser Dialysierflüssigkeit wurde bestimmt und mit der Menge multipliziert. Daraus ergab sich die Gesamtmenge an entfernter Substanz. Dieses Verfahren ist jedoch für die Routine zu unpraktisch, da über 100 l Dialysierflüssigkeit gesammelt, gewogen und gemischt werden müssen. Schließlich hat sich herausgestellt, daß Harnstoff von Bakterien schnell metabolisiert wird, verbrauchte Dialysierflüssigkeit gewöhnlich stark bakteriell kontaminiert ist und somit die gemessene Harnstoffkonzentration nicht den wahren Wert wiedergibt.

Um das Problem der großen Flüssigkeitsmenge zu beseitigen, hat man vorgeschlagen, lediglich einen Teil der verbrauchten Dialysierflüssigkeit aufzufangen. Dieses Verfahren wird Partielle Dialysatsammlung, englisch partial dialysate collection genannt. Dabei wird etwa 1% der verbrauchten Dialysierflüssigkeit kontinuierlich gesammelt. Am Ende wird die aufgefangene Menge gewogen, gemischt und eine Probe entnommen, die analysiert wird. Die in der Probe gefundene Konzentration wird mit der Menge multipliziert und durch den Anteilsfaktor (Verhältnis aus Gesamtdialysat/gesammelten Dialysat) multipliziert. Daraus ergibt sich die gesamte entfernte Menge. Mit diesem Verfahren beseitigt man das Mengenproblem, nicht jedoch das der bakteriellen Kontamination. Man kann nun die zur Sammlung bestimmte Teilmenge an verbrauchter Dialysierflüssigkeit durch ein Sterilfilter in den Probenaufnahmebehälter leiten. Dies löst das Problem der bakteriellen Kontamination, allerdings unter Inkaufnahme erhöhter Kosten.

Mehrere Verfahren sind bekannt, um eine partielle Dialysatsammlung durchzuführen: Stiller und Schaefer (Stiller Siegfried, Schaefer Udo, inventors. Stiller Siegfried, assignee. Passiver Dialysatfluss-Teiler. DE patent 3312909. 10/18/84) beschreiben einen passiven Flußteiler bei dem der gesamte Dialysatfluß durch eine Vielzahl von Kapillaren geleitet wird. Über eine oder mehrere Kapillaren wird ein Teilstrom zur partiellen Dialysatsammlung entnommen. Nachteil dieser Anordnung ist, daß schon geringfügige Veränderungen der Strömung das Teilerverhältnis verändern können, ohne daß dies bemerkbar wird. Ähnliches trifft auf eine Anordnung zu, bei der eine Kanüle in den Dialysatfluß eingeführt wird (Ing TS, YU AW, Khalaf MN, Tiwari P, Rafiq M, Khan AA, Nawab ZM. Collection of Hemodialysate Aliquot Whose Composition Reflects That of Total Dialysate. ASAIO Abstracts 1994;:85).

Eine andere Anordnung (Aviram A, Peters JH, Gulyassy PF. Dialysance of Amino Acids and Related Substances. Nephron 1971;8:440-54) arbeitet mit einer Pumpe, die einen Teilstrom aus dem Dialysatstrom entnimmt. Diese Anordnung hat den Nachteil, daß der Dialysatfluß konstant gehalten werden oder aber die Rate der Teilstrompumpe entsprechend nachgeführt werden muß.

Beide Verfahren haben den Nachteil, die Probe aus einem Bereich des Hydrauliksystems zu entnehmen, der üblicherweise bakteriell kontaminiert ist und in Geräten, deren Sterilisation oder Desinfektion in Rezirkulation erfolgt, nie desinfiziert bzw. sterilisiert wird.

Hämodialysegeräte müssen von Zeit zu Zeit, üblicherweise wenigstens einmal am Tag desinfiziert werden. Sie werden laufend neu kontaminiert: Durch unsteriles Wasser, unsteriles Dialysekonzentrat aber auch durch die Anschlußprozedur von Dialysatoren, die dialysierflüssigkeitsseitig üblicherweise nicht aseptisch erfolgt. In einigen Geräten erfolgt die Desinfektion durch Erhitzung des zuströmenden Wassers auf mehr als 85 Grad C oder durch Beimischung eines Desinfektionsmittels. Das Hitzeverfahren wird dabei zunehmend bevorzugt, da es ohne potentiell umweltgefährdende Desinfektionsmittel auskommt. Es hat jedoch den Nachteil, daß Keime im Wasser wegen der kurzen Durchlaufzeit nicht vollständig abgetötet werden. Sofern also die Wasserversorgung kontaminiert ist, wird das Gerät laufend neu kontaminiert.

Einige Geräte verfügen über Dialysatfilter, die unmittelbar vor dem Dialysator das Dialysat sterilfiltrieren. Diese müssen ihrerseits gespült werden, was üblicherweise während des Desinfektionsvorganges erfolgt. Dadurch geraten Keime am Dialysatoranschluß vorbei zur Abflußleitung, die somit rekontaminiert wird.

Es sind deshalb Geräte entwickelt worden, die eine laufende Rekontamination dadurch verhindern, daß nach einer initialen Spül- und Reinigungsphase das Gerät so geschaltet wird, daß Wasser bzw. Desinfektionslösung im Gerät rezirkuliert. Dadurch wird das Gerät sowohl vom Zufluß als auch vom Abfluß getrennt und kann nicht mehr rekontaminiert werden. Eine solche Anordnung ist z.B. in der DE 3447989 (Polaschegg HD, inventors. Fresenius AG, assignee. Hämodialysevorrichtung. DE patent 3447989, 7/16/87) beschrieben. Die Zugabe des Desinfektionsmittels kann dabei über einen Desinfektionsmittelbehälter erfolgen, der in den Rezirkulationskreislauf eingefügt wird, wie z.B. in der DE 4138140 (Polaschegg HD, inventors. Fresenius AG, assignee. Vorrichtung zur Desinfektion von Hämodialysegeräten mit einem pulverförmigen Konzentrat. DE patent 4138140. 12/23/93) beschrieben.

Nachteilig ist dabei, daß die Abflußleitung, aus der üblicherweise der Teilstrom zur Probennahme entnommen wird, nicht desinfiziert wird.

Die Druckschrift FR 2,692,983 A1 beschreibt die Eichung von von Meßfühler-Paaren, die zum Messen von chemischen oder physikalischen Größen einer Dialyseflüssigkeit verwendet werden. Dabei ist eine Messsensor am Einlass (Probennahmeventil) und der andere Messsensor am Auslass (Auslassventil) eines Dialysekreislaufes angeordnet. Eine wechselseitige Schaltung des Probennahmeventils und des Auslassventils ist dieser Druckschrift jedoch nicht zu entnehmen.

Die vorliegende Erfindung hat zur Aufgabe, die oben beschriebenen Nachteile zu vermeiden.

Das erfindungsgemäße Verfahren erlaubt, eine partielle Dialysatsammlung kontaminationsarm mit einem Hämodialysegerät durchzuführen, das mit einem Rezirkulationsverfahren desinfiziert bzw. sterilisiert wird.

Dies erfolgt dadurch, daß der Rezirkulationszweig des Hämodialysegerätes zur Probennahme aufgetrennt wird und über eine Ventilschaltung ein Teilstrom der verbrauchten Dialysierflüssigkeit entnommen wird.

Eine weitere Aufgabe der Erfindung ist die Angabe von Steuerungsverfahren zur Probenentnahme mit Hilfe zweier Ventile.

Gelöst werden diese Aufgaben durch Verfahren zur Steuerung einer Vorrichtung zur partiellen Dialysatsammlung mit einem Hämodialysegerät, das mit einem Auslassventil (110) und einem Probennahmeventil (120) versehen ist und das sich dadurch auszeichnet, dass die Ventile (110, 120) wechselseitig geöffnet bzw. geschlossen werden, wobei das Schalten der Ventile (110, 120) in vorgebbaren Probennahmezeitintervallen erfolgt und die Öffnungszeit des Probennahmeventils (120) vorgebbar ist.

Vorzugsweise erfolgt die Steuerung von Auslaß- und Probennahmeventil im festen Zeitintervallverhältnis zueinander.

In einer bevorzugten Ausführungsform erfolgt die Steuerung des Probennahmeventils in Abhängigkeit vom Dialysatvolumen, das im vorhergegangenen Auslaßventilöffnungsintervall verarbeitet wurde.

Darüber hinaus kann die Öffnungszeit des Probennahmeventils aus dem im vorhergegangenen Auslaßventilöffnungsintervall verarbeiteten Dialysatvolumen berechnet werden.

In einer bevorzugten Ausführungsform ist die Volumenmeßeinrichtung eine Bilanzkammer, wobei die Steuerung von Auslaß- und Probennahmeventil im Verhältnis ganzzahliger Bilanzkammervolumina erfolgt.

Des weiteren können parallel zum Probennahmeventil weitere Ventile mit Leitungen und Steckverbindungen vorgesehen werden.

Besondere Ausführungsformen des Hämodialysegerätes sind durch wenigstens einen Sensor gekennzeichnet, der die Verbindung der Leitung(en) vom/ von den Probennahmeventil(en) mit dem Eingang des Hämodialysegerätes detektiert.

Vorzugsweise sind die Verbindungsstücke zwischen der Probennahmeleitung (121) einerseits und dem Probenaufnahmebehälter (124) bzw. dem Eingangsbehälter des Dialysegerätes (13) andererseits so ausgeführt, daß zwischen dem Ausgang der Probennahmeleitung (122) und dem Eingangsbehälter des Hämodialysegerätes (13) ein Behälter mit Desinfektionsbzw. Reinigungskonzentrat eingefügt werden kann.

Die Erfindung ist an Hand der folgenden Abbildungen näher beschrieben.

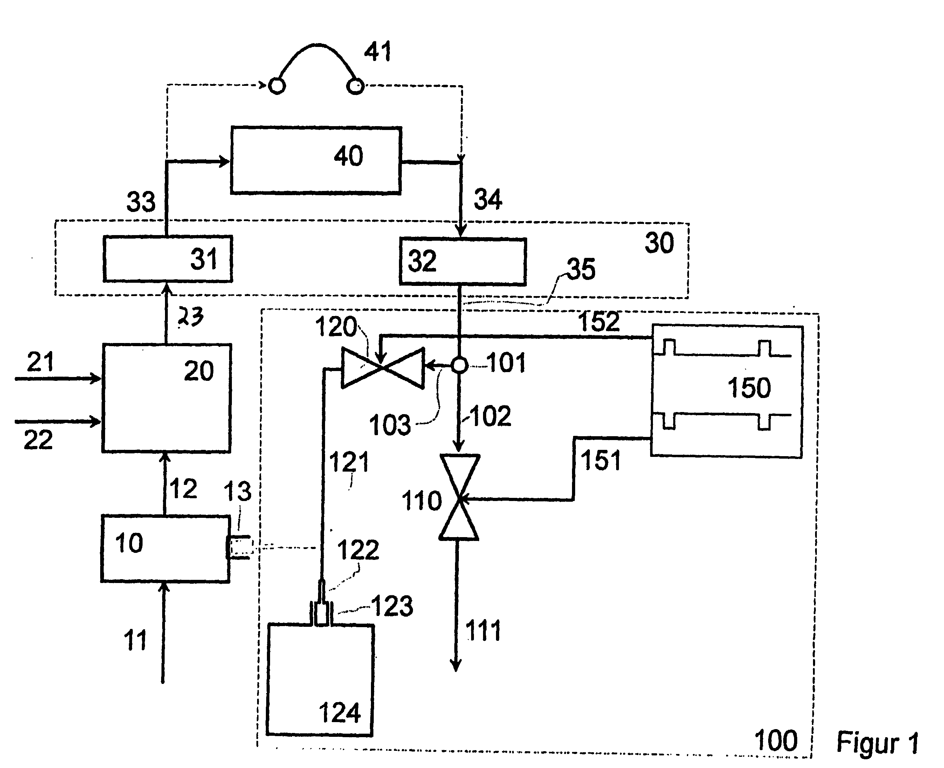

Fig 1 zeigt schematisch das Fließschema eines Dialysegerätes, das eine erfindungsgemäß einsetzbare Einrichtung enthält.

*Wassereingang:*

Für die Hämodialyse geeignetes Wasser von einer nicht näher dargestellten Wasserquelle gelangt über die Leitung 11 in den Eingangsbehälter 10. Von dort führt eine Verbindungsleitung 12 zur Dialysataufbereitungseinheit 20. Mit 13 ist eine Steckverbindung bezeichnet, die die Verbindung einer Rezirkulationsleitung (103, 120, 121, 122) mit dem Wassereingangsbehälter 10 erlaubt.

*Dialysataufbereitung:*

Der Dialysataufbereitungseinheit wird Hämodialysekonzentrat über die Leitungen 21 und 22 zugeführt. Üblicherweise handelt es sich dabei um sogenanntes Säurekonzentrat bzw. Bicarbonatkonzentrat. Statt zweier Konzentrate kann aber auch ein einziges Konzentrat oder auch mehr als 2 Konzentrate zugeführt werden. Im letzteren Fall sind mehr Leitungen erforderlich. In der Dialysataufbereitungseinheit wird Dialysat gemischt, erwärmt und entgast. Über die Leitung 23 gelangt das Dialysat in die Flüssigkeitsbilanziereinheit 30.

*Flüssigkeitsbilanzierung:*

Bei der Flüssigkeitsbilanziereinheit kann es sich um ein System basierend auf Bilanzkammern oder um ein System basierend auf Flußmessern handeln. Solche Systeme sind u.a. in der Arbeit des Erfinders: "Polaschegg HD. Methoden und Geschichte der Ultrafiltrationskontrolle in der Hämodialyse. Wiss. Info. Fresenius Stiftung Aktuelle Nephrologie 1985;18:135-49" beschrieben.

31 ist die Flußmeß- oder Kontrollvorrichtung für das frische Dialysat. Von dort gelangt das frische Dialysat über Leitung 33 und eine nicht näher bezeichnete Steckverbindung zum Dialysator 40. Alternativ kann Leitung 33 mit dem Kurzschlußstück 41 verbunden werden, was die Reinigung und Desinfektion des Hämodialysegerätes nach Beendigung der Dialyse ermöglicht. Vom Dialysator 40 bzw Kurzschlußstück 41 gelangt die, im Falle der Dialyse verbrauchte Dialysierflüssigkeit über die Leitung 34 zur ausgangseitigen Flußmeß- oder Kontrollvorrichtung 32 und von dort über Leitung 35 zur Probennahme- und Rezirkulationseinheit 100. Nicht dargestellt sind Überwachungs- und Schutzvorrichtungen zur Überwachung der richtigen Zusammensetzung der Dialysierflüssigkeit bzw. der Funktion der Flüssigkeitsbilanziervorrichtung.

*Probennahmevorrichtung:*

Die Probennahme- und Rezirkulationseinrichtung 100 besteht aus einem Abzweigstück 101, das über die Leitung 35 mit verbrauchter Dialysierflüssigkeit gespeist wird. Von dort gelangt die Dialysierflüssigkeit entweder über Leitung 102, Absperrventil 110 und Leitung 111 zum Abfluß oder alternativ über Leitung 103 zu Absperrventil 120. Vom Ventil 120 führt eine Leitung 121 zur Steckverbindung 122. Die Steckverbindung 122 ist mit der Steckverbindung 13 verbindbar, wodurch ein Rezirkulationskreislauf zur Desinfektion bzw. Sterilisation herstellbar ist. Alternativ kann Steckverbindung 122 mit Steckverbindung 123 verbunden werden. In diesem Zustand ist eine Sammlung von Dialysatproben im, mit der Steckverbindung 123 verbundenen Probensammelbehälter 124 möglich. Zwischen Steckverbindung 122 und 13 ist ein Behälter mit einem flüssigen oder festen Desinfektionsmittelkonzentrat einfügbar, wie er z.B. im Prospekt CleanCart™ der Firma Gambro, Lund, Schweden beschrieben ist.

Bei dem Probensammelbehälter handelt es sich bevorzugt um einen flexiblen Beutel aus einem Material mit geringer Gasdurchlässigkeit. Das Aufnahmevermögen liegt typisch bei 2 l, was etwa 2% der im Laufe einer Dialysebehandlung verbrauchten Dialysierflüssigkeit entspricht. Ventil 110 und 120 werden von der Steuereinrichtung 150 gesteuert.

Dabei ist die Steuereinrichtung 150 über die Verbindung 151 mit dem Auslaßventil 110 und über die Verbindung 152 mit dem Probennahmeventil 120 verbunden.

Sollte keine Probennahme gewünscht werden, so bleibt Ventil 120 geschlossen und Ventil 110 geöffnet. Zur Probennahme wird nun in zeitlich gleichen Abständen das Ventil 110 geschlossen während das Ventil 120 geöffnet wird. Die jeweilige Öffnungszeit der beiden Ventile ist so bemessen, daß das Verhältnis dem gewünschten Verhältnis von Probenmenge zu Gesamtmenge an Dialysierflüssigkeit entspricht. Die bevorzugte Probenmenge beträgt 1 - 2% .Die Häufigkeit der Probennahme sollte mindestens drei mal pro Dialyse betragen und ist nach oben hin nur durch die Schaltgeschwindigkeit der Ventile begrenzt. Bevorzugt liegt die Schalthäufigkeit zwischen 30 und 100 pro Dialyse. Für eine Dialysedauer von 3 h (180 min) ergeben sich somit für eine Schalthäufigkeit von 30/Dialyse Intervalle von 6 Minuten, wobei das Probennahmeventil 120 jeweils für 7,2 Sekunden (2% der Zeit) geöffnet wird.

*Desinfektion/Sterilisation:*

Zur Desinfektion bzw. Sterilisation wird, wie bereits beschrieben, der Stecker 122 mit der Steckverbindung 13 verbunden. Für eine Periode von typisch 5 Minuten nach Beginn des Desinfektionsvorganges bleibt Ventil 110 geöffnet und Ventil 120 geschlossen um das Dialysegerät von Dialysierfüssigkeit freizuspülen. Danach wird Ventil 110 geschlossen und Ventil 120 geöffnet. Gleichzeitig wird in nicht näher dargestellter Weise auch die Wasser- und Konzentratzuführ zum Dialysegerät unterbrochen. Das im Dialysegerät befindliche Wasser, eventuell mit einem Desinfektionsmittel versetzt und/oder erhitzt, rezirkuliert nun in bekannter Weise durch das Hämodialysegerät, wodurch dieses desinfiziert oder sterilisiert wird.

Die Ausgestaltung der Erfindung betrifft die Steuerung der Ventile 110 (Auslaßventil) und 120 (Probennahmeventil).

Im folgenden wird als Probennahmeintervall der Zeitraum vom Öffnen des Auslaßintervalls bis zum darauffolgenden Öffnen des Auslaßventils bezeichnet. Das Probennahmeintervall beginnt somit mit dem Öffnen des Auslaßventils, enthält das Schließen des Auslaßventils und endet mit dem Wiederöffnen des Auslaßventils. Das Probennähmeventil ist zu Beginn geschlossen, wird dann geöffnet und zu Ende des Probennahmeintervalls wieder geschlossen.

Die Steuerung des Probennahmeventils bzw. Auslaßventils kann rein zeitabhängig erfolgen, wie bereits beschrieben. Um bei größeren Zeitintervallen der Probennahme Schwankungen des Dialysatflußes oder der Druckverhältnisse auszugleichen, kann die Steuerung auch volumenabhängig erfolgen. Dazu wird in die Dialysatleitung vor dem Verzweigungspunkt 101 ein Flußmesser eingefügt und für jedes Zeitintervall die Öffnungsdauer des Probennahmeventils (120) so gesteuert, daß die Probenmenge dem vorgegebenen Anteil an der Gesamtmenge im Probennahmeintervall entspricht. Nimmt im Probennahmeintervall der Fluß zu, was bedeutet, daß das verarbeitete Dialysatvolumen zunimmt, so wird das Probennahmeventil für eine kürzere Zeit geöffnet, um das Volumenverhältnis gleich zu halten. Die Öffnungszeit kann gleichfalls durch den Volumensensor kontrolliert werden. In diesem Fall werden also die Ventile 110 und 120 so gesteuert, daß das Volumenverhältnis dem vorgegebenen entspricht, wobei das Zeitintervall vorgegeben ist. Die Steuerung bewirkt, daß die Probennahme zwar im festen Zeitintervall erfolgt, das Probennahmevolumen aber dem im Öffnungszeitintervall des Auslaßventiles verarbeiteten Dialysatvolumen angepaßt wird.

Sollte das Hämodialysegerät über eine volumetrische Flüssigkeitsbilanziereinheit erfolgen, so kann die Volumenmeßvorrichtung für verbrauchtes Dialysat (32) als Meßvorrichtung zur Steuerung der Ventile 110 und 120 herangezogen werden.

Alternativ kann die Steuerung der Ventile 110 und 120 aber auch so erfolgen, daß das Probennahmeventil jeweils dann geöffnet wird, wenn eine vorherbestimmte Dialysatmenge erreicht wird.

Verfügt das Hämodialysegerät über eine volumetrische UF-Kontrolle mit Bilanzkammern, wie sie z. B. im Deutschen Patent: "Schäl W, inventors. Fresenius AG, assignee. Vorrichtung zur Hämodialyse und zum Entziehen von Ultrafiltrat. DE patent 2838414. 10/31/84" beschrieben ist, so kann die Bilanzkammerschaltung zur Steuerung der Probennahme herangezogen werden. Dazu wird das Probennahmeventil synchron mit jeder n-ten Bilanzkammerumschaltung geschaltet. n ist dabei das Teilerverhältnis, das, bei einem Füllvolumen der Bilanzkammer von 30 ml bei typisch 30 - 100 liegt.

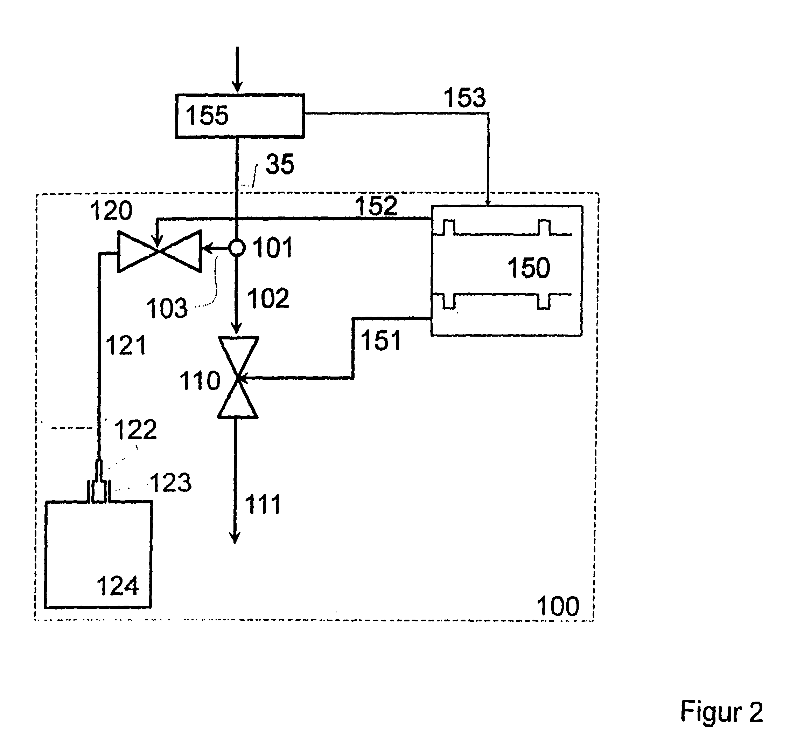

Die beschriebenen Ausgestaltungen sind in den Figuren 2 und 3 sowie 4a-c näher dargestellt. Es zeigt Figur 2 einen Ausschnitt aus Figur 1. Hinzugefügt ist eine Leitung 153, die von einer Flußmeßvorrichtung (155) für das verbrauchte Dialysat zur Steuervorrichtung 150 führt. Sollte das Hämodialysegerät über eine Flüssigkeitsbilanziervorrichtung mit Flußmessern verfügen, so kann diese Einrichtung ebenfalls verwendet werden. In diesem Fall ist die Flußmeßvorrichtung 155 identisch mit der Meßvorrichtung für verbrauchtes Dialysat 32.

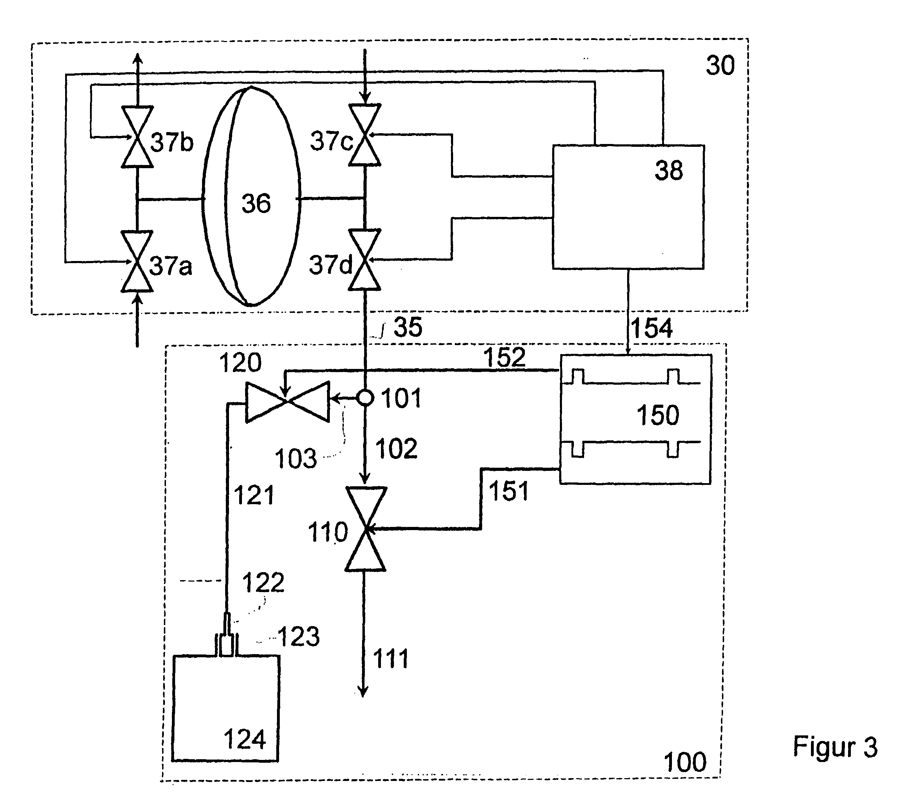

Figur 3 zeigt einen Ausschnitt aus Figur 1 mit einer Flüssigkeitsbilanziervorrichtung 30, die mit Hilfe einer Bilanzkammer arbeitet. Statt einer einzigen können auch zwei oder mehrere Bilanzkammern verwendet werden. Die Flüssigkeitsbilanziervorrichtung 30 besteht in diesem Fall aus einer Bilanzkammer 36 und den vier Ventilen 37 a-d, die von der Steuereinrichtung 38 gesteuert werden. Diese steuert die Ventile 37 so, daß während einer Füllphase jeweils die Ventile 37a und d geöffnet und die Ventile 37b und c geschlossen sind. Aus diesem Zustand wird die Bilanzkammer in die Zirkulationsphase geschaltet, wobei die Ventile 37b und c geöffnet und die Ventile 37a und d geschlossen werden. Von der Bilanzkammersteuereinrichtung 38 führt eine Signalleitung 154 zur Probennahmesteuereinrichtung 150.

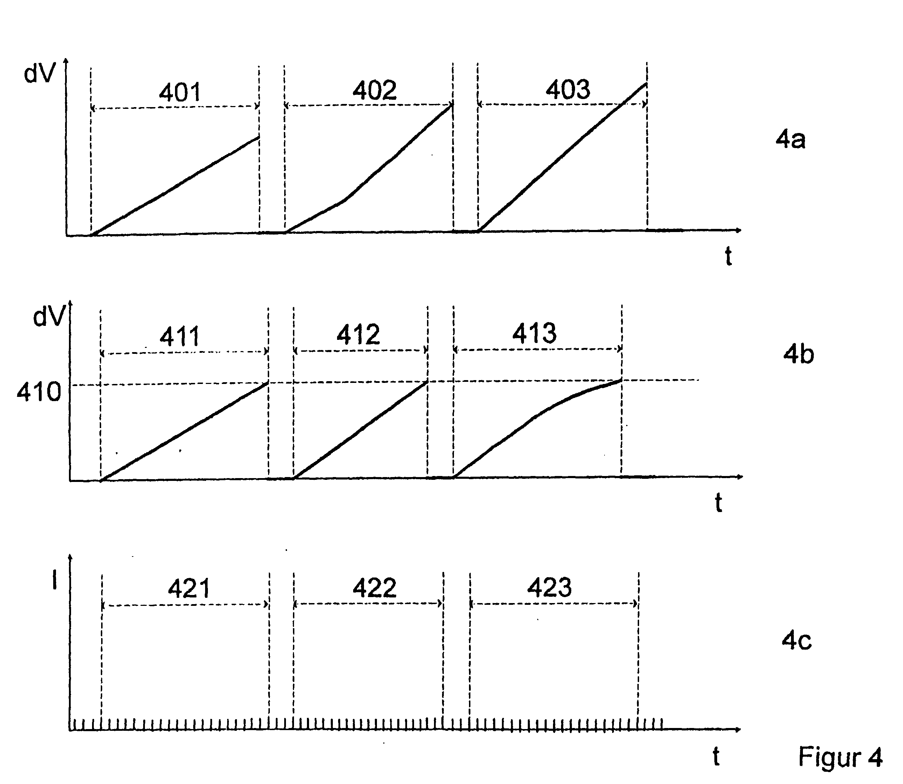

Figur 4 a bis c zeigt schematisch die Wirkungsweise der Probennahmesteuereinrichtung 150 für die weiter oben beschriebenen Ausgestaltungen der Erfindung die in Figur 2 und 3 dargestellt sind.

Figur 4a zeigt die Steuerung des Probennahmeventils 120 mit Hilfe der in Figur 2 dargestellten Anordnung, bei der ein Flußmesser zur Messung des verbrauchten Dialysats verwendet wird. Im Diagramm ist die Zeitachse mit t bezeichnet. dV stellt das im Zeitintervall durch das Auslaßventil 110 strömende Volumen dar. Das Auslaßventil 110 bleibt dabei jeweils für ein vorgegebenes Zeitintervall geöffnet. Die Öffnungszeitintervalle 401, 402, 403 für das Auslaßventil (110) sind somit gleich groß. In der Figur werden sie durch strichlierte Linien begrenzt. In den dazwischenliegenden Intervallen wird das Auslaßventil 110 geschlossen und das Probennahmeventil 120 geöffnet. Sollte sich innerhalb eines Öffnungszeitintervalls des Auslaßventils der Fluß ändern, so wird die Schließzeit des Ventils 110 bzw. Öffnungszeit des Ventils 120 entsprechend angepaßt. In der Figur 4a ist im Intervall 401 der Fluß konstant, was zu einer linearen Zunahme des verarbeiteten Volumens dV führt. Im Intervall 402 nimmt das Volumen nichtlinear zu, der Fluß steigt gegen Ende an. Da der Fluß am Ende des Intervalls größer als der mittlere Fluß im Intervall ist, wird die darauffolgende Öffnungszeit des Probennahmeventils 120 so gesteuert, daß das Volumenverhältnis Probenmenge/Gesamtmenge verbrauchtes Dialysat dem vorgegebenen entspricht. Im vorliegenden Fall führt dies zu einer Verkürzung der Öffnungszeit.

Die Berechnung des Öffnungsintervalls erfolgt dabei nach folgendem Schema:

Es sei V das im Öffnungszeitintervalls des Auslaßventils geförderte Volumen, q(T) der zeitabhängige Fluß des verbrauchten Dialysats am Ende des Intervalls T wobei der zeitliche Beginn des Intervalls jeweils zu 0 gesetzt wird. tp ist die Öffnungszeit des Probennahmeventils und F das vorgegebene Verhältnis von Probenmenge zur Gesamtmenge an Dialysierflüssigkeit, z.B. 0,03.

$$tp = \frac{V}{q(T)} * \frac{F}{1-F} \qquad \text{Formel (1)}$$

Alternativ kann die Öffnungszeit des Probennahmeventils durch den Flußmesser gesteuert werden. Das Probennahmeventil wird so lange geöffnet, bis die Probenmenge Vp gleich dem vorgegebenen Anteil F an der Gesamtmenge ist. Die im Öffnungszeitintervall des Auslaßventils geförderte Menge entspricht dabei 1-F. Figur 4b zeigt eine weitere Alternative der Regelung der Öffnungszeit des Probennahmeintervalls. Das Auslaßventil wird jeweils geschlossen und das Probennahmeventil geöffnet, wenn eine vorherbestimmte Menge an verbrauchter Dialysierflüssigkeit (z.B. 1 l) erreicht wird. Diese Menge ist in der Figur mit 410 bezeichnet. Schematisch zeigt die Figur 4b für das Intervall 411 einen konstanten Fluß, für das Intervall gleichfalls einen konstanten, jedoch gegenüber 411 größeren Fluß und für das Intervall 413 einen abnehmenden Fluß. Dementsprechend sind die Zeitintervalle 411, 412, 413 unterschiedlich lang. Die Öffnungszeit des Probennahmeventils kann entweder nach Formel (1) berechnet oder aber durch den Flußmesser wie schon beschrieben geregelt werden.

Figur 4c zeigt eine Alternative zur Steuerung der Öffnungszeiten der Ventile, falls eine Volumenmeßvorrichtung mit Bilanzkammern verwendet wird. Über der Zeitachse sind dabei die Bilanzkammerumschaltimpulse als kurze Striche aufgetragen. Nach einer vorgegebenen Anzahl von Impulsen ( im Beispiel sind es 20) wird das Auslaßventil für eine ebenfalls vorgegebene Anzahl an Impulsen (im Beispiel 3) geschlossen bzw. das Probennahmeventil geöffnet. Da ein Bilanzkammertakt jeweils einem bestimmten Volumen entspricht ergibt sich somit ein Verhältnis für das Probenvolumen und Gesamtvolumen, das unabhängig vom Fluß ist und dem Verhältnis F entspricht. Im Beispiel ist F = 3/23. Die Probennahmeintervalle sind dabei vom Fluß abhängig. In Figur 4c ist beispielhaft angenommen, daß der Fluß im Intervall 422 zunimmt. Die Abstände der Bilanzkammerimpulse sind geringer und das Intervall 422 insgesamt kürzer als die Nachbarintervalle 421 und 423.

Die Methode der partiellen Dialysatsammlung erlaubt die Berechnung der Menge an entfernter Substanz, sie gibt aber keine Auskunft über die Kinetik diese Vorganges, d.h. ob der Abfall der Konzentration über die Zeit z.B. einem 1-pool oder 2-pool Modell entspricht. Durch die Verwendung von zusätzlichen Probennahmeventilen, die parallel zum beschriebenen geschaltet werden, ist es möglich, einzelne Proben zu Beginn und zum Ende der Dialysebehandlung zu gewinnen. Daraus oder in Kombination mit dem über die gesamte Dialysedauer gesammelten Dialysatprobe lassen sich dann Aussagen über die Kinetik des Vorganges machen. An Stelle von mehreren Ventilen kann auch eine umschaltbare Probennahmevorrichtung nachgeschaltet werden. Diese kann entweder ihrerseits mehrere Ventile enthalten oder aber eine Auffangbehälterwechselvorrichtung.

Als Probenauffangbehälter kann sowohl ein starres Gefäß als auch ein Beutel verwendet werden. Ein steriler oder sterilisierbarer Behälter ist bevorzugt. Besonders bevorzugt ist ein Beutel, der durch ein Septum verschlossen ist und aus einem Material mit geringer Gasdurchlässigkeit besteht. Die geringe Gasdurchlässigkeit verhindert, daß $CO_2$ aus dem Dialysat entweicht, was zu einer Veränderung des pH Wertes führen würde. Ein Septum als auffangbehälterseitige Steckverbindung (123) ist besonders kostengünstig. Die geräteseitige Steckverbindung 122 besteht dann bevorzugt aus einem Dorn, wie er von Infusionsleitungen her bekannt ist, der aus einer verriegelbaren Steckverbindung herausragt. Die Kupplung (13) am Wassereingang besteht dann aus einem Gegenstück zu dieser verriegelbaren Steckverbindung. Eine verriegelbare Steckverbindung ist z.B. eine Luer-Lock Verbindung, wie sie in der Medizintechnik verbreitet ist. Die Kupplung 13 kann auch noch ein Rückschlagventil oder einen automatischen Verschlußmechanismus enthalten, der die Öffnung verschließt, wenn der Stecker 122 nicht mit der Kupplung 13 verbunden ist. An der Kupplung 13 kann ferner ein Sensor angebracht sein, der die Verbindung des Steckers 122 mit der Kupplung 13 zu detektieren erlaubt. Wird während einer Hämodialysebehandlung durch diesen Sensor eine Verbindung von Stecker 122 und Kupplung 13 detektiert, so wird das Probennahmeventil unabhängig von einem laufenden Probennahmeprogramm geschlossen und das Auslaßventil geöffnet, das Probennahmeprogramm also abgebrochen oder der Start eines Probennahmeprogramms verhindert. Dadurch wird vermieden, daß es während einer Hämodialysebehandlung zu einer Rezirkulation verbrauchten Dialysats kommt. Während der Desinfektion in Rezirkulation bleibt das Probennahmeventil 120 geöffnet und das Auslaßventil 110 geschlossen. Das Desinfektionsprogramm kann nur gestartet werden, wenn die Verbindung von Stecker 122 und Kupplung 13 durch den oben genannten Sensor detektiert wurde. Für den Fall, daß während der Desinfektion die Lösung dieser Verbindung durch den Sensor detektiert wird, wird das Desinfektionsprogramm abgebrochen und das Probennahmeventil 120 geschlossen.

Als Sensor kann z.B. ein mechanischer Mikroschalter, ein magnetischer oder optischer Sensor eingesetzt werden.

## Patentansprüche

1. Verfahren zur Steuerung einer Vorrichtung zur partiellen Dialysatsammlung mit einem Hämodialysegerät, das mit einem Auslassventil (110) und einem Probennahmeventil (120) versehen ist, **dadurch gekennzeichnet, dass** die Ventile (110, 120) wechselseitig geöffnet bzw. geschlossen werden, wobei das Schalten der Ventile (110, 120) in vorgebbaren Probennahmezeitintervallen erfolgt und die Öffnungszeit des Probennahmeventils (120) vorgebbar ist.

2. Verfahren nach Anspruch 1, **dadurch gekennzeichnet, dass** man das Probennahmezeitintervall aus der Gesamtzeit der Dialysebehandlung und der vorgebbaren Probenanzahl berechnet.

3. Verfahren nach Anspruch 1 und 2, **dadurch gekennzeichnet, dass** man die Öffnungszeit des Probennahmeventils (120) aus dem Verhältnis des vorgebbaren Gesamtprobenvolumens zum Gesamtdialysatvolumen multipliziert mit dem Probennahmezeitintervall berechnet.

4. Verfahren nach Anspruch 1, **dadurch gekennzeichnet, dass** das Schalten der Ventile (110, 120) in einem festen Zeitintervall erfolgt, wobei die Öffnungszeit des Probennahmeventils aus der im Auslassventilöffnungsintervall gemessenen Dialysatmenge errechnet wird.

5. Verfahren nach Anspruch 1, **dadurch gekennzeichnet, dass** die verbrauchte Dialysatmenge im Auslassventilöffnungsintervall mit einem Durchflussmesser gemessen wird und das Schalten der Ventile (110, 120) erfolgt, sobald ein vorgegebenes Volumen an verbrauchter Dialysierflüssigkeit erreicht ist.

6. Verfahren nach Anspruch 5, **dadurch gekennzeichnet, dass** als Durchflussmesser eine Bilanzkammer verwendet wird und die Umschaltung der Ventile (110, 120) erfolgt, sobald eine vorgegebene Anzahl von Bilanzkammertakten erreicht ist.

## Claims

1. Procedure for the control of a device for partial dialysate collection with a piece of haemo-dialysis equipment which is provided with a discharge valve (110) and sample-taking valve (120), **characterised in that** the valves (110, 120) are alternately opened or closed whereby the switching of the valves (110, 120) takes place at pre-determined intervals between successive sampling stages and the time that the sample-taking valve (120) remains open is pre-determined.

2. Procedure in accordance with claim 1, **characterised in that** the time interval between successive sampling stages is calculated from the total duration time of the dialysis treatment and the pre-determinable number of samples taken.

3. Procedure in accordance with claims 1 and 2, **char-**

**acterised in that** the time the sample-taking valve (12) remains open is calculated from the ratio of the pre-determinable total volume of the samples to the total dialysate volume multiplied by the time interval between successive sample-taking stages.

4. Procedure in accordance with claim 1, **characterised in that** the switching of the valves (110, 120) takes place at a fixed time interval, whereby the time the sampling valve remains open is calculated from the amount of dialysate measured during the interval between the successive openings of the discharge valve.

5. Procedure in accordance with claim 1, **characterised in that** the amount of dialysate used in the interval between successive openings of the discharge valve is determined by a flow meter and the switching of the valves (110, 120) takes place as soon as a predetermined volume of used dialysis liquid has been measured.

6. Procedure in accordance with claim 5, **characterised in that** a balance chamber is used as flow meter and the reverse switching of the valves (110, 120) takes place as soon as a predetermined number of balance chamber cycles has been completed.

**Revendications**

1. Procédé de réglage d'un appareil pour le recueil partiel de dialysat à l'aide d'un appareil d'hémodialyse, doté d'une valve de desserrage (110) et d'une valve de prélèvement (120), **caractérisé en ce que** les valves (110, 120) sont ouvertes ou fermées de façon bidirectionnelle, où le branchement des valves (110, 120) a lieu à des intervalles de prélèvement prédéfinissables, et le temps d'ouverture de la valve de prélèvement (120) est prédéfinissable.

2. Procédé selon la revendication 1, **caractérisé en ce que** l'on calcule l'intervalle de prélèvement à partir de la durée totale de la dialyse et du nombre prédéfinissable d'échantillons.

3. Procédé selon les revendications 1 et 2, **caractérisé en ce que** la durée d'ouverture de la valve de prélèvement (120) est calculée à partir du rapport du volume total d'échantillon prédéfinissable/volume total du dialysat, multiplié par l'intervalle de prélèvement d'échantillon.

4. Procédé selon la revendication 1, **caractérisé en ce que** le branchement des valves (110, 120) a lieu à intervalles fixes, où la durée d'ouverture de la valve de prélèvement est déterminée à partir de la quantité de dialysat calculée durant l'intervalle d'ouverture de la valve de desserrage.

5. Procédé selon la revendication 1, **caractérisé en ce que** la quantité de dialysat consommée dans l'intervalle d'ouverture de la valve de desserrage est mesurée à l'aide d'un débitmètre, et que le branchement des valves (110, 120) a lieu dès qu'un volume prédéfini est atteint pour le liquide de dialyse utilisé.

6. Procédé selon la revendication 5, **caractérisé en ce qu'**une chambre de mesure a fonction de débitmètre, et que le branchement des valves (110, 120) a lieu dès que l'on atteint un nombre prédéfini de cycles de la chambre de mesure.

Figur 1

Figur 2

EP 1 053 759 B1

Figur 3

EP 1 053 759 B1

EP 1 053 759 B1

dV

401 402 403

4a

t

dV

411 412 413

4b

410

t

I

421 422 423

4c

t Figur 4